# EUROPEAN PATENT APPLICATION

(11) **EP 2 893 950 A1**
(43) Date of publication of application: **15.07.2015**
(21) Application number: 14151172.5
(22) Date of filing: 14.01.2014
(51) Int. Cl.: A61M 25/00, B29C 47/02, B29C 59/00

(54) **Catheter and method for manufacturing such catheter**

(71) Applicant: Alvimedica Vascular Research B.V., 9403 PA Assen (NL)
(72) Inventor: Heiner, Wilfred Peter, 9403 PA Assen (NL); Schutten, Bert Jan, 7921 VM Zuidwolde (NL)
(74) Representative: Verdijck, Gerardus

(57) **Abstract**

The present application relates to a catheter and a method for manufacturing such catheter. The catheter (2) comprising a tubular structure having a proximal and (14) and a distal end (12), the tubular structure comprising a coil structure (24) and having an outer surface and an inner surface defining a lumen, wherein the inner surface comprises a number of micro-bumps (32).

## Description

The present invention relates to a catheter specifically including a so-called micro-catheter, preferably a single-lumen catheter that can be inserted into the vasculature using a guide wire.

Catheters are known from practice and comprise a flexible tube that is used for entering the vasculature and other body parts without requiring extensive diagnostic or surgical operations.

Problems relating to conventional catheters, including micro-catheters, involve the delivery of agents and devices, including instruments, through relatively small size vessels for cardiovascular use thereby causing damage to vessels that may cause serious health problems.

The present invention has as one of its objectives to provide a catheter that reduces the risk of damaging a vessel.

This objective is achieved with a catheter according to the invention, the catheter comprising a tubular structure having a proximal and a distal end, the tubular structure comprising a coil structure and having an outer surface and an inner surface defining a lumen, wherein the inner surface comprises a number of micro-bumps.

In the context of the present invention catheters include guiding catheters, micro-catheters and other catheters. In a presently preferred embodiment according to the invention the catheter specifically relates to a micro-catheter, and more specifically to a single-lumen micro-catheter. This catheters can be used for cardiovascular use, for example.

By providing a tubular structure having a proximal end and a distal end, and with the tubular structure comprising a coil structure, the tubular structure acts as a reinforcement structure of the catheter. Preferably, the coiled structure is made of multiple wires that are wrapped around a carrier and are then compressed to a solid structure. The multiple wires form a closed spring structure without any significant spacing between the individual wires of one layer. The spring structure acts with behavior similar to a compression element. The coil structure may comprise a single layer or alternatively multiple layers, for example 2, 3, 4, 5, 6 or more layers in a tight spring element construction with adjacent layers being preferably wound clockwise and anti-clockwise. Alternatively, the structure is made out of a metal tube involving a laser cut process such that similar behavior is achieved. The tubular structure forms a stable base for the (micro) catheter.

The inner surface of the catheter, more specifically the inner surface of the tubular structure thereof, defines a lumen through which material can be delivered to a specific location. According to the invention the inner surface comprises micro-bumps. By providing micro-bumps an irregular surface structure for the inner surface of the catheter is achieved. Such micro-bumps involve grooves, relief, embossments, crater-like wells or other irregular elements. Surprisingly, providing such micro-bumps in the lumen of the catheter reduces the surface contact of the catheter with elements, such as a guide wire that is inserted in the lumen, thereby reducing the friction forces. This reduction significantly reduces the risk of damaging the surroundings of the catheter when inserting a guide wire in the lumen, for example. Therefore, the micro-bumps are preferably provided over a substantial length of the tubular structure between the proximal and distal end thereof. More specifically, the micro-bumps are preferably provided over at least 50% of the length between the distal and proximal ends.

Preferably, the micro-bumps have a depth in the range of 0.1 - 50 µm, more preferably 0.5 - 40 µm, and most preferably 1 - 30 µm. Using wires to provide the (coil or) tubular structure, involves winding a layer of the tubular structure out of 6 to 20 wires with an individual diameter of 0.025 - 0.2 mm. After the formation of the coil structure some irregularities between adjacent windings result. These irregularities result in micro-bumps in the aforementioned specific range. For example, a wire with a diameter of about 0.7 mm will result in a micro-bump of about approximately 10 µm. In a presently preferred embodiment according to the present invention the micro-bumps typically have a spiraled or partial-spiraled shape.

To effectuate the positive effect the micro-bumps have on the friction reduction the coating and/or laminating layers on the inner surface of the tubular structure, if any, should be relatively thin. Preferably, a coating is directly applied to the coiled tubular structure, preferably directly applied to the metal thereof, without a laminated polymer layer. Providing a relatively thin coating and/or laminating layer results in the grooves or dips between individual windings of the tubular structure acting as micro-bumps in accordance with the invention.

Preferably, the outside of the tubular structure is laminated with polymers of different grades, thereby providing the possibility to provide an additional stiffness gradient over the length of the (micro) catheter.

In a preferred embodiment according to the present invention the coil structure comprises welds connecting together a number of windings over single layer and/or of multiple layers.

By connecting together windings of a single layer and/or of multiple layers the catheter can be tuned according to the intended use of the (micro) catheter. For example, for cardiovascular use different operations require differently tuned reinforcement structures. In one of the embodiments according to the present invention such tuning is achieved by using spot welding to connect windings of the spring-type tubular structure together. Increasing the number of spot welds will increase the stiffness of the tubular structure. Also, when connecting more layers together the stiffness of the structure will increase. The amount of layers that is connected together can be manipulated by choosing the amount of power that is used to make a spot weld. These welds can be made over the length of the tubular structure thereby defining a welding structure that in a presently preferred embodiment is non-homogeneous over the length of the tubular structure. The welding structure may involve spot welds at random positions and/or with some structure. The welding structure may relate to providing a straight line, a spiraled line or a waved line of welds. It is noted that in the context of the present invention welding relates to welding and soldering.

For example, most catheters should be more flexible at the distal end and more stiff at the proximal end to guarantee a more or less predictable behavior of especially the distal tip of the catheter. Therefore, the welds at the proximal end will be stronger and preferably provided in a larger number as compared to the distal end thereof. This tuning of the tubular structure by providing a welding structure provides a tuned catheter that is relatively easy to apply while reducing the risk of causing damage. In addition, the catheter according to the present invention can be manufactured in an relatively effective manner.

In another presently preferred embodiment according to the present invention the tubular structure further comprises a multi-layer coiled structure with at the distal end a reduced number of layers as compared to the proximal end of the catheter.

By taking away one or more layers of wires of the coiled tubular structure a tapered reinforcement tube is achieved that will be more flexible towards the distal end of the catheter with the (partly) removed or not applied layers. Preferably, At the location where a layer ends this end is preferably welded to the layer directly beneath it to enable transfer of forces and/or energy between these layers through the welded connections. This further enhances the possibilities for tuning of the tubular structure and dedicating the catheter to its specific use. Preferably, the manipulation of the number of layers is applied in combination with providing a welding structure to enhance the flexibility of tuning the catheter.

The invention further relates to a method for manufacturing a catheter, the method comprising the steps of:
- providing a tubular structure having a proximal end and a distal end; and
- providing an inner surface layer on an inner surface of the tubular structure with the inner surface layer comprising micro-bumps.

This method provides the same effects and advantages as those mentioned in relation to the catheter.

In a presently preferred embodiment the method relates to the manufacture of a so-called micro-catheter having a coiled tubular structure with the inner surface layer thereof comprising micro-bumps that provide a friction reduction as earlier described in relation to the catheter.

In a further embodiment the method step of providing an inner surface layer comprises a laminating step and/or a coating step to provide a lubricious layer on the inner surface of the tubular structure.

By providing an inner surface layer as a lubricious layer the friction forces can be further reduced.

When laminating a polymer layer the polymer material is preferably modified so that it has specific properties to provide a lubricious surface. For example, this can be achieved applying doping and/of functional nanoparticles. When applying a (almost) pure polymer to create an inner liner for the catheter an additional lubricious coating will preferably be applied. The lamination process for an inner layer preferably involves inserting an additional tubular structure. This tubular structure may relate to a small tube that is closed on one end and connected to a precious source on the other end. When heating the cold tube locally the inner tube will inflate at that specific location and heat-seal against the inner surface of the relatively cold tube. Preferably, the inner layer is calibrated by pulling a hot bean or another hot element through the lumen of the catheter. This assures that the adhesion is good and, in addition, that the lumen of the catheter has approximately the same diameter over substantially the whole length of the catheter.

When coating the inner surface of the tubular structure with a lubricious coating sufficient adhesion of the coating to the metal material of the tubular structure can be achieved by applying heat curing coating. This can be performed with an optional pre-etching of the inside of the tubular structure. Optionally, the coating may involve a multiple layer system and/or a multiple component system. According to the invention the laminating and/or coating steps maintain the micro-bumps to reduce the friction forces and prevent damage caused by the catheter.

In a presently preferred embodiment according to the invention the manufacturing of a catheter comprises the step of reinforcing the coil structure of the tubular structure by locally welding together windings of the coil structure. Therefore, windings of a single layer and/or multiple layers of the tubular structure are welded together. In a presently preferred embodiment according to the present invention this involves spot welding resulting in a welding structure preferably resulting in a spiralled or waved welding line. This enables tuning of the tubular structure depending on the actual intended use of the catheter. In addition, or as an alternative thereto, one or more windings of the multi-layer tubular structure are locally removed. As mentioned before for the catheter, this is preferably done at the distal end of the catheter to provide a flexible distal end. It is noted that in context of the present invention locally removing of (part of) the windings may relate to not providing windings at a specific location.

Further advantages, features and details of the invention are elucidated on the bases of preferred embodiments thereof, wherein reference is made to the accompanying drawings, in which:
- Fig. 1 shows a schematic overview of a catheter according to the invention;
- Fig. 2 shows details in a cross-section of the micro-bumps as provided in the catheter of fig. 1;
- Fig. 3 shows a tuned distal end of the catheter of Fig. 1; and
- Fig. 4 shows the manufacturing steps of the catheter of Fig. 1-3.

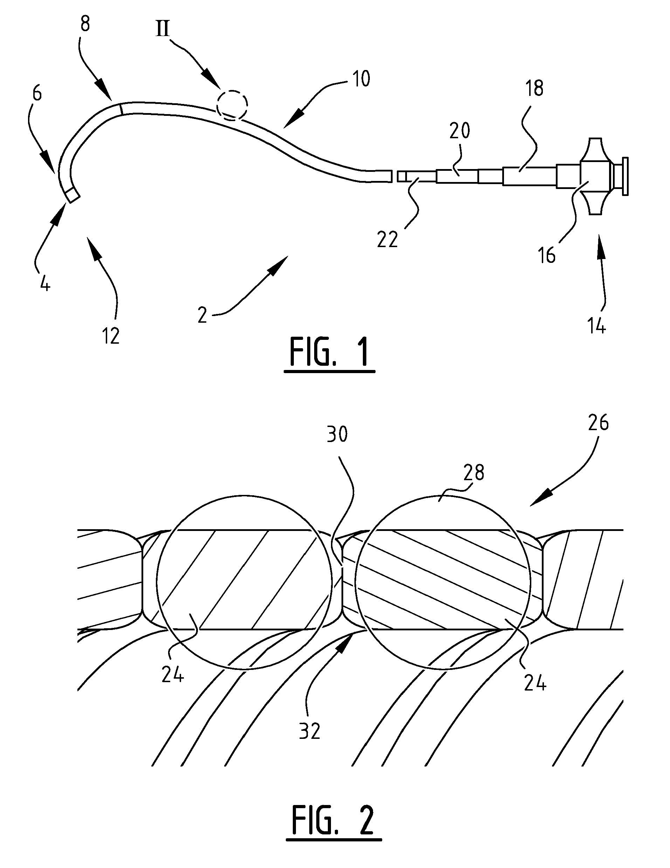

Catheter 2 (Fig. 1) comprises a soft tip 4, a tertiary distal part 6, a secondary intermediate part 8 and a primary proximal part 10. In the illustrated embodiment the three different parts 6, 8, 10 of catheter 2 are provided with increasing stiffness as seen from distal end 12 to proximal end 14. At proximal end 14 catheter 2 comprises hub 16. Furthermore, catheter 2 comprises a strain relief 18, a reinforcement structure 20 and a Teflon liner 22.

In the illustrated embodiment catheter 2 (Fig. 2) comprises a tubular/coil structure having metallic helical hollow strands with a number of windings 24. Preferably, a layer of coil structure 24 comprises 6 to 20 wires/strands. Coil structure 24 comprises a number of layers. When manufacturing catheter 2 the tubular structure starts with structure 26 having a number of windings 28 that are compressed to engage each other with contact surface 30. In the illustrated embodiment, adjacent contact surface 30 between two adjacent windings 24, micro-bump 32 is formed. In the illustrated micro-catheter the wires that are used have a diameter in the range of 0.025-0.2 mm. It will be understood that the invention can also be applied to guiding catheters which may use wires with a diameter in a range of 0.07 - 0.1 mm. In the illustrated embodiment the use of a wire of 0.07 mm diameter will approximately provide a micro-bump of about 10 µm in catheter 2.

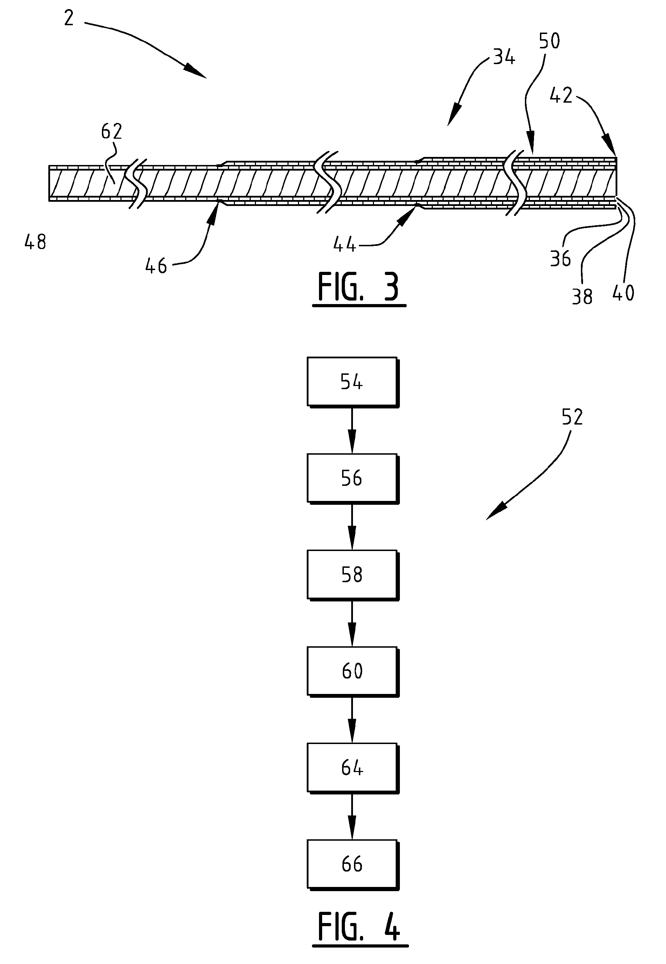

In the illustrated embodiment catheter 2 (Fig. 3) relates to a multi-layer tubular structure 34 with an inner layer 36, intermediate layer 38 and an outer layer 40. Preferably, adjacent layers are winded in opposite direction, i.e. clockwise or counter-clockwise. In fact, this provides multi-wire tubular structure 34 with multiple layers 36, 38, 40 of a tight winded spring construction in clockwise and counter-clockwise layers. Structure 34 acts as reinforcement tube of (micro)-catheter 2.

In the illustrated embodiment at (local) welding spot 42 the three layers are joined together with a smooth weld or soldering connection. Such local spot 42 tunes the characteristics of catheter 2. Towards distal end 12 of catheter 2 at first reduction point 44 outer layer 40 is welded or soldered to intermediate layer 38. At second reduction point 46 intermediate layer 38 is welded or soldered to inner layer 36. The number of layers and location of reduction points 44, 46 tunes the characteristics of catheter 2. In the illustrated embodiment the distance between first and second reduction points 44, 36 may amount to 280 mm. It will be understood that other distances will also be possible in accordance with the invention.

At the inside of coiled structure 36 coating 48 is provided. At the outside of coil structure 34 a laminar layer 50 is provided.

When manufacturing catheter 2 (Fig. 4), in manufacturing process 52 a coil structure manufacturing step 54 is performed that in the illustrated embodiment starts with helical hollow strands. These strands can be made of different materials, including Pt alloys or Nitinol. It will be understood that other materials can also be applied in accordance with the present invention.

In a first tuning step 56 layers 38, 40 can be removed locally. The ends of a layer are soldered or welded to the layer directly underneath resulting in a tapered configuration of catheter 2. The connection between different layers 36, 38, 40 enables forces/energy to be transferred to other layers.

In a second tuning step 58 windings in a single layer and/or of multiple layers are connected by welding or soldering to define a welding structure.

In laminating or coating step 60 lumen 62 of catheter 2 is provided with a laminating layer and/or coating layer 48. This involves heat curing and optionally pre-edging of the inside of inner layer 36 of coil structure 34, for example. In case of a lamination process a small tube will be inserted in lumen 62. In the illustrated embodiment this tube will be closed on one end and will be connected to a pressure source on the other end to heat-seal to the inner surface of coil structure 34. To calibrate inner layer 48 a hot bean can be pulled through lumen 62 to assure both a good adhesion and a substantially similar diameter over substantially the whole length of catheter 2.

Next, lamination process step 62 is performed for outer layer 50 of catheter 2 involving a heating process. This may involve locally heating coil structure 34 and providing a pressure to the laminating outer tube close to this location such that outer layer 50 will laminate with outer layer 40 of coil structure 34. The outer laminating tube can be a pre-assembled tube or it can be laminated using a heat shrink tube that is removed after the lamination process.

Liner 22 will be connected to the proximal end of tubular structure 34 in connecting step 66 and will be connected to hub 16 involving a hub over moulding process. In the illustrated embodiment, liner 22 is laminated inside the tube.

It will be understood that sections and layers of catheter 2 can be modified with different materials to adjust characteristics of catheter 2. Layer 50 on the outside of catheter 2 will assure that the surface will be a closed polymer surface to prevent thrombus on the surface.

The present invention is by no means limited to the above described and preferred embodiments thereof. The rights sought are defined by the following claims wherein the scope of which many modifications can be envisaged.

## Claims

1. Catheter comprising a tubular structure having a proximal and a distal end, the tubular structure comprising a coil structure and having an outer surface and an inner surface defining a lumen, wherein the inner surface comprises a number of micro-bumps.

2. Catheter according to claim 1, wherein the micro-bumps are provided over a substantial length of the tubular structure.

3. Catheter according to claim 1 or 2, wherein the micro-bumps have a depth in the range of 0.1-50 µm, preferably in the range of 0.5-40 µm, and more preferably in the range of 1-30 µm.

4. Catheter according to claim 1, 2 or 3, wherein the micro-bumps having a spiralled shape.

5. Catheter according to one or more of the claims 1-4, wherein the coil structure comprising welds connecting together a number of windings of a single layer and/or of multiple layers.

6. Catheter according to claim 5, wherein the welds define a welding structure that is non-homogeneous over the length of the tubular structure.

7. Catheter according to one or more of the foregoing claims, the tubular structure further comprising a multi-layer coil structure with at the distal end a reduced number of layers as compared to the proximal end.

8. Method for manufacturing a catheter, comprising the steps of:
- providing a tubular structure from a coil structure, with the tubular structure having a proximal end and a distal end; and
- providing an inner surface layer on an inner surface of the tubular structure with the inner surface layer comprising micro-bumps.

9. Method according to claim 8, wherein providing an inner surface layer comprises a laminating step and/or a coating step to provide a lubricious layer on the inner surface of the tubular structure.

10. Method according to claim 9, wherein the laminating comprises:
- inserting an additional tubular structure; and
- locally heating the tubular structure of the catheter such that the additional tubular structure locally heat-seals with the inner surface of the tubular structure of the catheter.

11. Method according to claim 9 or 10, further comprising the step of calibrating the inner layer, wherein the calibrating preferably comprises pulling a calibration element through the lumen.

12. Method according to one or more of the claims 8-11, further comprising the step of reinforcing the coil structure of the tubular structure by locally welding together windings of the coil structure, wherein windings of a single layer are welded together and/or wherein the windings of more than one layer are welded together.

13. Method according to one or more of the claims 8-12, wherein the welding comprises spot welding.

14. Method according to one or more of the claims 8-13, wherein the welding comprises line welding, wherein the line welding preferably results in a spiraled or waved welding line.

15. Method according to one or more of the claims 8-14, wherein one or more windings of the multi-layer tubular structure are locally removed.
